# EUROPÄISCHE PATENTSCHRIFT

(11) **EP 1 322 162 B1**
(45) Veröffentlichungstag und Bekanntmachung des Hinweises auf die Patenterteilung: **22.03.2006**
(21) Anmeldenummer: 01980402.0
(22) Anmeldetag: 18.09.2001
(51) Int. Cl.: A01N 47/44, A01N 33/12, A01N 33/04

(54) **DESINFEKTIONSMITTEL**
DISINFECTANT AGENT
AGENT DE DESINFECTION

(30) Priorität: 20.09.2000 EP 00120586
(43) Veröffentlichungstag der Anmeldung: 02.07.2003
(73) Patentinhaber: Lonza AG, 4052 Basel (CH)
(72) Erfinder: LICHTENBERG, Florian, 79639 Grenzach-Wyhlen (DE); LÜTZELER, Michael, 79639 Grenzach-Wyhlen (DE); RANFT, Volker, D-79730 Murg (DE)
(86) Internationale Anmeldenummer: PCT/EP2001/010755
(87) Internationale Veröffentlichungsnummer: WO 2002/023992

(56) Entgegenhaltungen:
- EP-A- 0 860 117
- DE-A- 3 606 294
- US-A- 4 601 954
- DATABASE WPI Section Ch, Week 198528 Derwent Publications Ltd., London, GB; Class A97, AN 1985-168483 XP002190272 & JP 60 097908 A (KURITA WATER IND LTD), 31. Mai 1985 (1985-05-31)

## Beschreibung

Die Erfindung betrifft synergistische Desinfektionsmittelzusammensetzungen auf Basis von Aminen.

Es sind zahlreiche Desinfektions- und Konservierungsmittelzusammensetzungen auf Basis von Aminen und/oder quartären Ammoniumsalzen bekannt. Diese weisen jedoch im allgemeinen, insbesondere bei höheren Verdünnungen, eine unbefriedigende Wirksamkeit gegen Pilze wie z. B. *Aspergillus niger* auf.

EP-A-0 860 117 beschreibt Reinigungs- und/oder Desinfektionsmittel auf Basis von quartären Ammoniumsalzen organischer Säuren, die ein Biguanid und/oder Guanidiniumacetat enthalten können. Über die Wirksamkeit gegen Pilze wird nichts offenbart.
US-A-4 601 954 beschreibt Desinfektionsmittel auf Basis von quartären Ammoniumchloriden und Harnstoff oder Guanidinhydrochlorid.

Aufgabe der vorliegenden Erfindung war daher die Bereitstellung von Desinfektionsmittelzusammensetzungen auf Basis von Aminen, welche auch bei hoher Verdünnung eine gute Wirksamkeit gegen Pilze aufweisen.

Erfindungsgemäss wird diese Aufgabe durch die Desinfektionsmittelzusammensetzung nach Patentanspruch 1 gelöst.

Es wurde überraschend gefunden, dass Amine der allgemeinen Formel 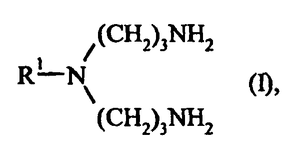 worin R¹ C₆₋₁₈-Alkyl bedeutet,
durch Zusatz von wenigstens einem Guanidiniumsalz der Formel 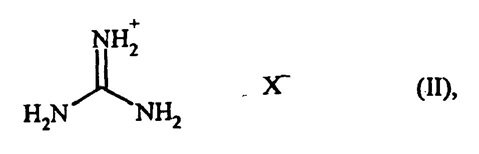 worin X⁻ ein einwertiges Anion oder ein Äquivalent eines mehrwertigen Anions einer anorganischen oder organischen Säure bedeutet,
im Massenverhältnis (I):(II) von 20:1 bis 1:20 eine gute fungizide Wirksamkeit erhalten. Unter Alkyl sind hier und im folgenden jeweils lineare oder verzweigte Alkylgruppen der angegebenen Kohlenstoffzahlen zu verstehen, vorzugsweise jedoch lineare Alkylgruppen und besonders bevorzugt solche mit gerader Zahl von Kohlenstoffatomen. Insbesondere sind hierunter auch die von natürlichen Rohstoffen abgeleiteten Homologengemische wie beispielsweise "Kokosalkyl" zu verstehen.

Unter substituiertem Phenyl sind insbesondere mit einer oder mehreren C₁₋₈Alkylgruppen und/oder Chloratomen substituierte Phenylgruppen zu verstehen.

Das Amin (I) ist vorzugsweise *N*,*N-*Bis(3-aminopropyl)dodecylamin, *N*,*N*-Bis(3-aminopropyl)octylamin, oder ein Gemisch dieser Verbindungen.

Das Anion X⁻ des Guanidiniumsalzes ist vorzugsweise Chlorid, Bromid, Rhodanid, Nitrat, Hydrogensulfat, Sulfat, Dihydrogenphosphat, Monohydrogenphosphat, Phosphat, Carbonat oder Acetat. Es ist auch möglich, ein Gemisch zweier oder mehrerer solcher Salze einzusetzen.

Besonders gute Ergebnisse wurden mit Guanidiniumcarbonat [(CH₆N₃)₂CO₃] erzielt.

Das Massenverhältnis von Amin (I) zu Guanidiniumsalz (II) liegt vorzugsweise im Bereich von 1:5 bis 5:1.

Die erfindungsgemässen Desinfektionsmittelzusammensetzungen enthalten vorzugsweise Wasser als Lösungsrnittel, gegebenenfalls in Kombination mit einem organischen . Lösungsmittel.

Vorzugsweise enthalten die erfindungsgemässen Desinfektionsmittelzusammensetzungen noch einen oder mehrere Hilfsstoffe aus der Gruppe bestehend aus organischen Lösungsmitteln, Tensiden, Komplexbildnem, Duftstoffen und Farbstoffen.

Ein bevorzugtes Anwendungsgebiet der erfindungsgemässen Desinfektionsmittelzusammensetzungen ist die Flächen- und Instrumentendesinfektion.

Weitere bevorzugte Anwendungsgebiete sind die Wäschedesinfektion und die Händedesinfektion.

Die erfindungsgemässen Desinfektionsmittelzusammensetzungen eignen sich auch gut für den Einsatz in chemischen Toiletten wie beispielsweise an Bord von Flugzeugen und Fahrzeugen.

Ein weiteres bevorzugtes Einsatzgebiet ist die Konservierung von technischen Flüssigkeiten wie beispielsweise Wasserkreisläufe bei der Papierherstellung, Kühlwasser, Bandschmiermittel für Transportbänder oder Kühlschmierstoffe bei der Metallbearbeitung.

Eine ebenfalls bevorzugte Anwendung ist schliesslich der Einsatz als Schutz- und Konservierungsmittel für organische oder biologisch angreifbare Konstruktionsmaterialien wie beispielsweise Holz.

Die folgenden Beispiele verdeutlichen die Ausführung der Erfindung. Alle Mengenangaben sind, soweit nicht anders angegeben, in Massen-%. Als Testkeim wurde jeweils *Aspergillus niger* ATCC 16404 eingesetzt.

### Beispiel 1

Es wurde eine Desinfektionsreinigerformulierung (Konzentrat) hergestellt aus:

| | |
|---|---|
| 5,0% | Didecyldimethylammoniumchlorid (50%ige Lösung) |
| 2,0% | *N,N*-Bis(3-aminopropyl)dodecylamin |
| 5,0% | Guanidiniumcarbonat |
| 5,0% | Genapol® T250 (Talgfettalkoholpolyglycolether, 25 mol Ethylenoxid) |
| 0,5% | Natriummetasilicat |
| 0,25% | Natriumcarbonat |
| 3,0% | Ethylendiamintetraessigsäure-Tetranatriumsalz (40%ige Lösung) Wasser ad 100% |

Die Wirksamkeit wurde mit einer Verdünnung (I Teil Konzentrat, 99 Teile Wasser) bei 20 °C und 15 min Kontaktzeit bestimmt. Testkeim war *Aspergillus niger* ATCC 16404. Der dekadische Logarithmus der Keimzahlreduktion war 4,0.

### Vergleichsbeispiel 1

Es wurde wie in Beispiel 1 verfahren, jedoch mit dem Unterschied, dass das Guanidiniumcarbonat durch die gleiche Menge Wasser ersetzt wurde. Unter den gleichen Testbedingungen war die Formulierung praktisch unwirksam.

### Beispiel 2

Es wurde eine Desinfektionsmittelformulierung (Konzentrat) hergestellt aus:

| | |
|---|---|
| 4,9% | *N,N*-Bis(3-aminopropyl)dodecylamin |
| 4,0% | Guanidiniumcarbonat |
| 2,0% | Genapol® T250 (Talgfettalkoholpolyglycolether, 25 mol Ethylenoxid) |
| 5,0% | Hostapur® SAS 30 (C₁₃₋₁₇ sekundäre *n*-Alkansulfonsäure, Natriumsalz) |
| 2,0% | Ethylendiamintetraessigsäure-Tetranatriumsalz (40%ige Lösung) |
| 0,7% | Ethylendiamintetraessigsäure Wasser ad 100% |

Die Wirksamkeit wurde mit einer Verdünnung (1 Teil Konzentrat, 99 Teile Wasser) bei 20 °C und 15 nun Kontaktzeit bestimmt. Der dekadische Logarithmus der Keimzahlreduktion war 4,1.

### Beispiele 3-7

Es wurden wässrige Lösungen aus 0,5% Guanidiniumsalz (II) und 0,25% Amin (I) hergestellt und nach dem in CEN 1275 spezifizierten Verfahren gestestet. Die Ergebnisse sind in Tabelle 1 zusammengefasst..

**Tabelle 1**

| **Beispiel Nr.** | **Amin** | **Guanidiniumsalz** | **Ig Keimreduktion** |
|---|---|---|---|
| 3 | *N*,*N*-Bis(3-aminopropyl)dodecylamin | Carbonat | >4 4 |
| 4 | dto. | Acetat | 4,0 |
| 5 | dto. | Nitrat | 3,3 |
| 6 | dto. | Chlorid | 3,5 |
| 7 | dto. | Sulfat | 2,8 |

Zum Vergleich wurden alle in Tabelle 1 aufgeführten Verbindungen als Einzelsubstanzen in 0,5%iger Lösung getestet. Keine dieser Verbindungen wies eine ausgeprägte fungizide Wirkung auf (1g Keirnreduktion <2).

## Patentansprüche

1. Desinfektionsmittelzusammensetzung, enthaltend
a) wenigstens ein Amin der allgemeinen Formel 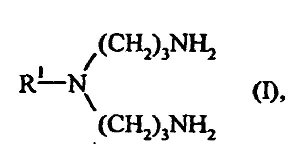 worin R¹ C₆₋₁₈-Alkyl bedeutet; und
b) wenigstens ein Guanidiniumsalz der Formel 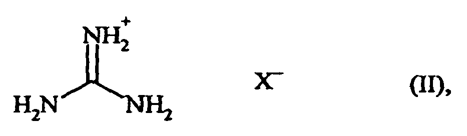 worin X⁻ ein einwertiges Anion oder ein Äquivalent eines mehrwertigen Anions einer anorganischen oder organischen Säure bedeutet,
im Massenverhältnis (I):(II) von 20:1 bis 1:20.

2. Desinfektionsmittelzusammensetzung nach Anspruch 1, **dadurch gekennzeichnet, dass** das Amin ausgewählt ist aus der Gruppe bestehend aus *N,N* Bis(3-aminopropyl)-dodecylamin und *N,N-*Bis(3-aminopropyl)octylamin sowie Gemischen dieser Verbindungen.

3. Desinfektionsmittelzusammensetzung nach Anspruch 1 oder 2, **dadurch gekennzeichnet, dass** X⁻ ausgewählt ist aus der Gruppe bestehend aus Chlorid, Bromid, Rhodanid, Nitrat, Hydrogensulfat, Sulfat, Dihydrogenphosphat, Monohydrogenphosphat, Phosphat, Carbonat und Acetat.

4. Desinfektionsmittelzusammensetzung nach einem der Ansprüche 1 bis 3, **dadurch gekennzeichnet, dass** das Massenverhältnis (I):(II) zwischen 1:5 und 5:1 liegt.

5. Desinfektionsmittelzusammensetzung nach einem der Ansprüche 1 bis 4, **dadurch gekennzeichnet, dass** sie Wasser als Lösungsmittel enthält.

6. Desinfektionsmittelzusammensetzung nach einem der Ansprüche 1 bis 5, **dadurch gekennzeichnet, dass** sie zusätzlich einen oder mehrere Hilfsstoffe aus der Gruppe bestehend aus organischen Lösungsmitteln, Tensiden, Komplexbildnern, Duftstoffen und Farbstoffen enthält.

7. Verwendung der Desinfektionsmittelzusammensetzungen gemäss Ansprüchen 1 bis 6 zur Flächen- und Instrumentendesinfektion.

8. Verwendung der Desinfektionsmittelzusammensetzungen gemäss Ansprüchen 1 bis 6 zur Wäschedesinfektion.

9. Verwendung der Desinfektionsmittelzusammensetzungen gemäss Ansprüchen 1 bis 6 zur Händedesinfektion.

10. Verwendung der Desinfektionsmittelzusammensetzungen gemäss Ansprüchen 1 bis 6 in chemischen Toiletten.

11. Verwendung der Desinfektionsmittelzusammensetzungen gemäss Ansprüchen 1 bis 6 als Konservierungsmittel für technische Flüssigkeiten.

12. Verwendung der Desinfektionsmittelzusammensetzungen gemäss Ansprüchen 1 bis 6 als Schutz- und Konservierungsmittel für Konstruktionsmaterialien.

## Claims

1. Disinfectant composition comprising
a) at least one amine of the general formula 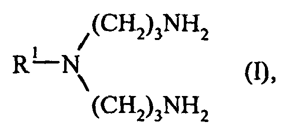 where R¹ is C₆₋₁₈-alkyl; and
b) at least one guanidinium salt of the formula 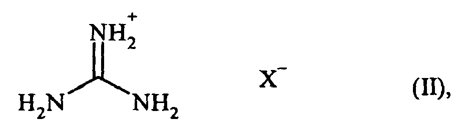 where X⁻ is a monovalent anion or one equivalent of a polyvalent anion of an inorganic or organic acid,
in the mass ratio (I):(II) of 20:1 to 1:20.

2. Disinfectant composition according to Claim 1, **characterized in that** the amine is selected from the group consisting of *N,N*-bis(3-aminopropyl)dodecylamine, *N,N*-bis(3-aminopropyl)-octylamine, and mixtures of these compounds.

3. Disinfectant composition according to Claim 1 or 2, **characterized in that** X⁻ is selected from the group consisting of chloride, bromide, rhodanide, nitrate, hydrogen sulfate, sulfate, dihydrogen phosphate, monohydrogen phosphate, phosphate, carbonate and acetate.

4. Disinfectant composition according to one of Claims 1 to 3, **characterized in that** the mass ratio (I):(II) is between 1:5 and 5:1.

5. Disinfectant composition according to one of Claims 1 to 4, **characterized in that** it comprises water as solvent.

6. Disinfectant composition according to one of Claims 1 to 5, **characterized in that** it additionally comprises one or more aids selected from the group consisting of organic solvents, surfactants, complexing agents, fragrances and colorants.

7. Use of the disinfectant compositions according to Claims 1 to 6 for surface disinfection and instrument disinfection.

8. Use of the disinfectant compositions according to Claims 1 to 6 for laundry disinfection.

9. Use of the disinfectant compositions according to Claims 1 to 6 for hand disinfection.

10. Use of the disinfectant compositions according to Claims 1 to 6 in chemical toilets.

11. Use of the disinfectant compositions according to Claims 1 to 6 as preservatives for industrial liquids.

12. Use of the disinfectant compositions according to Claims 1 to 6 as preservatives for construction materials.

## Revendications

1. Composition de désinfectant, contenant
a) au moins une amine de formule générale 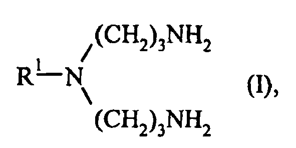 dans laquelle R¹ signifie alkyle en C₆ à C₁₈ ;
b) au moins un sel de guanidine de formule 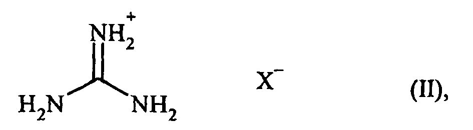 dans laquelle X⁻ est un anion monovalent ou un équivalent d'un anion polyvalent d'un acide inorganique ou organique,
dans un rapport massique (I):(II) de 20:1 à 1:20.

2. Composition de désinfectant selon la revendication 1, **caractérisée en ce que** l'amine est choisie dans le groupe constitué par la *N,N*-bis(3-aminopropyl)dodécylamine et la *N,N*-bis(3-aminopropyl)octylamine ainsi que les mélanges de ces composés.

3. Composition de désinfectant selon la revendication 1 ou 2, **caractérisée en ce que** X⁻ est choisie dans le groupe constitué par le chlorure, le bromure, le rhodanure, le nitrate, l'hydrogénosulfate, le sulfate, le dihydrogénophosphate, le monohydrogénophosphate, le phosphate, le carbonate et l'acétate.

4. Composition de désinfectant selon l'une quelconque des revendications 1 à 3, **caractérisée en ce que** le rapport massique (I): (II) est situé entre 1:5 et 5:1.

5. Composition de désinfectant selon l'une quelconque des revendications 1 à 4, **caractérisée en ce qu'**elle contient de l'eau comme solvant.

6. Composition de désinfectant selon l'une quelconque des revendications 1 à 5, **caractérisée en ce qu'**elle contient en outre un ou plusieurs adjuvants du groupe constitué par les solvants organiques, les agents tensioactifs, les complexants, les parfums et les colorants.

7. Utilisation de la composition de désinfectant selon les revendications 1 à 6 pour la désinfection de surfaces et d'instruments.

8. Utilisation de la composition de désinfectant selon les revendications 1 à 6 pour la désinfection du linge.

9. Utilisation de la composition de désinfectant selon les revendications 1 à 6 pour la désinfection des mains.

10. Utilisation de la composition de désinfectant selon les revendications 1 à 6 dans les toilettes chimiques.

11. Utilisation des compositions de désinfectant selon les revendications 1 à 6 comme conservateurs pour les liquides techniques.

12. Utilisation de la composition de désinfectant selon les revendications 1 à 6 comme agent de protection et conservateur pour les matériaux de construction.
